# EUROPEAN PATENT SPECIFICATION

(11) **EP 1 987 800 B1**
(45) Date of publication and mention of the grant of the patent: **21.04.2010**
(21) Application number: 08155072.5
(22) Date of filing: 24.04.2008
(51) Int. Cl.: A61F 2/01

(54) **Crossover Retrievable Medical Filter**
Abrufbarer medizinischer Crossover-Filter
Filtre médical extractible passif

(30) Priority: 01.05.2007 US 742790
(43) Date of publication of application: 05.11.2008
(73) Proprietor: Cordis Corporation, Miami Lakes, FL 33014 (US)
(72) Inventor: Fleming, James A. III, Bethlehem, Pennsylvania 18020 (US); Kinst, Thomas F., Hillsborough, New Jersey 08844 (US); Silver, James H., Palo Alto, California 94306 (US)
(74) Representative: Belcher, Simon James

(56) References cited:
- WO-A-2005/102439
- WO-A-2006/036457
- US-A1- 2006 069 406

## Description

The present invention relates to medical filters which are intended to be placed inside a blood vessel or other body passage for the purpose of intercepting thrombus or particles.

Medical filters, including vena cava filters, are generally known and used to intercept unwanted particulate material in blood vessels and the like. To function satisfactorily in a blood vessel, a filter must be effective to entrap thrombus, clots or other dangerous coagulations while allowing free flow of blood in the vessel. It has been found that certain features are desirable in such filters. It is important that the filter be emplaced with minimal trauma to the patient as by percutaneous delivery. It is also important that the filter be adapted to properly adjust to the size of the vessel. Of course, it is also important that the filter remain effective during its time in place. And, while the filter may be permanently emplaced, it is desirable if the filter can be easily retrieved if desired.

Generally speaking, vena cava and other medical filters are known. It is also known to deliver filters in compressed shape by means of a catheter and to remove such filters following their implantation. However, difficulties may be encountered with presently known filters, and there remains room for improved designs of filters and methods of delivery and removal of filters.

WO 2006 / 036457 discloses a medical filter with two baskets.

WO-A-2005/102439 relates to a long term medical filter. One example includes first and second filter sections connected by a central shaft. A hook is provided at one end. The filter sections comprise individual struts following a curving path.

Accordingly, the present invention provides a medical filter which is suitable for permanent implantation but can be removed at any time after implantation if desired. The filter of the present invention can be implanted and retrieved from either femoral or jugular approaches. In a preferred embodiment of the present invention, retrieval is facilitated by a crossover design of the filter baskets which minimizes the diameter of the filter during retrieval. The filter matches the clot capture efficiency of a double-basket filter yet is readily implanted and retrieved. Furthermore, the design practical for manufacture and can be economically made.

Thus, in accordance with the present invention, a medical filter for placement inside a body passage to treat a patient comprises:
in a radially compressed state, a longitudinally extending spine having a plurality of struts secured at one longitudinal end of said spine and a plurality of struts secured at an opposite longitudinal end of said spine, each of said struts extending generally longitudinally along said spine and each of said struts being biased in a radially direction; and
in a radially expanded state, each of said struts extending in a direction generally radially outwardly and axially from said spine and each of said struts having a first bend proximate to said spine in a direction away from said spine and a second bend at each free end portion in a direction toward said spine, as described in claim 1.

The present invention can be used in a method of retrieval of a medical filter comprising the steps of:
providing the medical filter inside a body passage of a patient,
withdrawing said filter into a lumen of a catheter by contacting each strut secured at one end of said spine with a distal tubular end of said catheter and bending each said strut radially inwardly while withdrawing a first part of said filter into said lumen; and then
continuing to withdraw said filter into said lumen by contacting each strut secured at said other end of said spine with said distal tubular end of said catheter thereby bending said struts in an inversely to a radially inward postion as said filter is withdrawn into said lumen.

Further understanding of the present invention will be had from the following description taken in conjunction with the accompanying drawings and appended claims.

Embodiments of the invention will now be described by way of example with reference to the accompanying drawings, in which:
FIG.1 is a perspective view of a preferred embodiment of a filter of the present invention shown in expanded form;
FIG. 2 is a longitudinal cross-sectional view of the filter of Figure 1 in place in a vein;
FIG. 3 is a side elevation view of the filter of Fig. 1 in compressed form and in an insertion cartridge;
FIG. 4 is a longitudinal cross-sectional view of the filter of Figure 1 in association with a insertion cartridge illustrating emplacement of the filter from the vein;
FIG. 5 is a longitudinal cross-sectional view of the filter of Figure 1 in association with a removal cartridge showing a step in removal of the filter from the vein;
FIG. 6 is a longitudinal cross-sectional view of the filter of Figure 1 in association with a removal cartridge showing a further step in removal of the filter from the vein;
FIG. 7 is a perspective view of an alternative preferred embodiment of a filter of the present invention;
FIG. 8 is a perspective view of another alternative preferred embodiment of a filter of the present invention;
FIG. 9 is a longitudinal cross-sectional view of the filter of Figure 8 in association with a removal cartridge and illustrating a step in retrieval of the filter of Figure 8; and
FIG. 10 is a longitudinal cross-sectional view of the filter of Figure 8 in association with a removal cartridge and illustrating a further step in retrieval of the filter of Figure 8.

The following description of the preferred embodiments of the present invention is intended to be merely illustrative in nature, and as such, is not intended to limit in any way the present invention, its application, or uses. Numerous modifications may be made by those skilled in the art without departing from the scope of the invention. For example, the preferred embodiments of the present invention are shown in use in a vein but may also be used in other body passages.

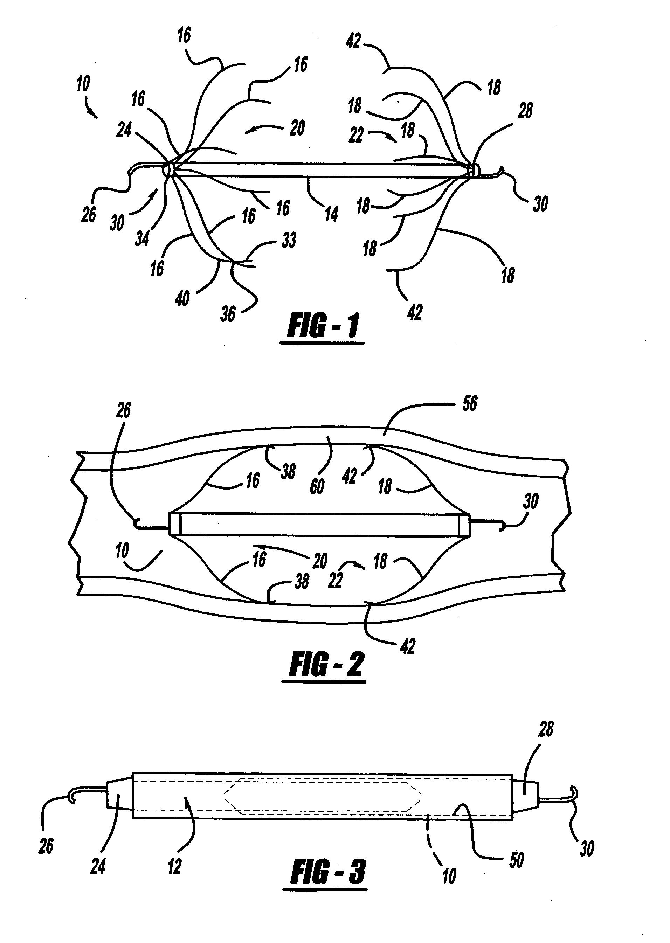

Now referring to Figures 1-3, a preferred embodiment of a medical filter of the present invention is shown and indicated generally by the numeral 10. Filter 10 is made of a resilient material which tends to expand to the form illustrated in Figure 1 but can be compressed to a smaller diameter form as shown in Figure 2 where Filter 10 is shown inside a suitable delivery catheter 12. Figure 3 shows filter 10 in place in a vein.

Filter 10 has a body 14 which extends generally along the longitudinal axis of filter 10 and which carries a plurality of radially and axially extending struts 16 and 18. Struts 16 combine to define a first filter basket 20 and struts 18 combine to define a second filter basket 22. A first collar 24 with retrieval hook 26 attached thereto is located at one longitudinal end of filter 10. A second collar 28 with retrieval hook 30 attached thereto is located at the other longitudinal end of filter 10. Filter 10 is symmetric in form although it will be appreciated by those skilled in the art that asymmetric forms of filter 10 are within the broad scope of the present invention. For example filter 10 may have more or less struts 16 than struts 18 and filter 10 may have a retrieval hook at one end but not the other end thereof. It is, however, preferred that filter 10 is symmetrical and has retrieval hooks at both ends to allow it to be retrieved using a snare either from a femoral or jugular approach.

Each strut 16 is attached to body 14 at a first end 32 thereof and is formed so as to extend, when not compressed, first generally axially along body 14 and then after bend 34 generally radially outwardly from body 14 for a distance slightly greater than the radius of a vena cava of intended use. The end portion 36 of each strut 16 is bent back slightly towards body 14 so that end 38 of strut 16 is radially inward with respect to bend radius 40.

Filter 10 may be made of any suitable material using a variety of methods. Nitinol and stainless steel are examples of suitable materials but other materials may be used so long as the material has the desired characteristics of strength, resilience, flexibility, biocompatibility and endurance and is suitable for the particular manufacturing technique employed. It is, of course, required that the material employed be capable of expanding to the desired shape upon ejection from the delivery catheter. Thus, the material must also be sufficiently resilient to accomplish both compression in the delivery catheter and expansion upon ejection from the catheter.

Suitable methods of manufacture include cutting a pattern into a tube to enable expansion of the tube into the desired body and struts. Another suitable method is forming the struts and body from separate strips or wires and then joining the respective parts together by suitable methods which are well known in the art.

Having described the structure of filter 10, a preferred embodiment of the present invention, further understanding of the unique character and advantages of the present invention will be had by an understanding of its use. Now referring to figures 2-6, filter 10 is intended to be initially deployed in the lumen 50 of a delivery catheter 12 as shown in Figure 3. Insertion of filter 10 into delivery catheter 12 can be by any conventional method including by simply pushing filter 10 into the lumen at the distal end of a delivery catheter. Alternatively, filter 10 may be inserted into the proximal end of a delivery catheter and pushed by means of a push wire or the like to the distal end of the catheter.

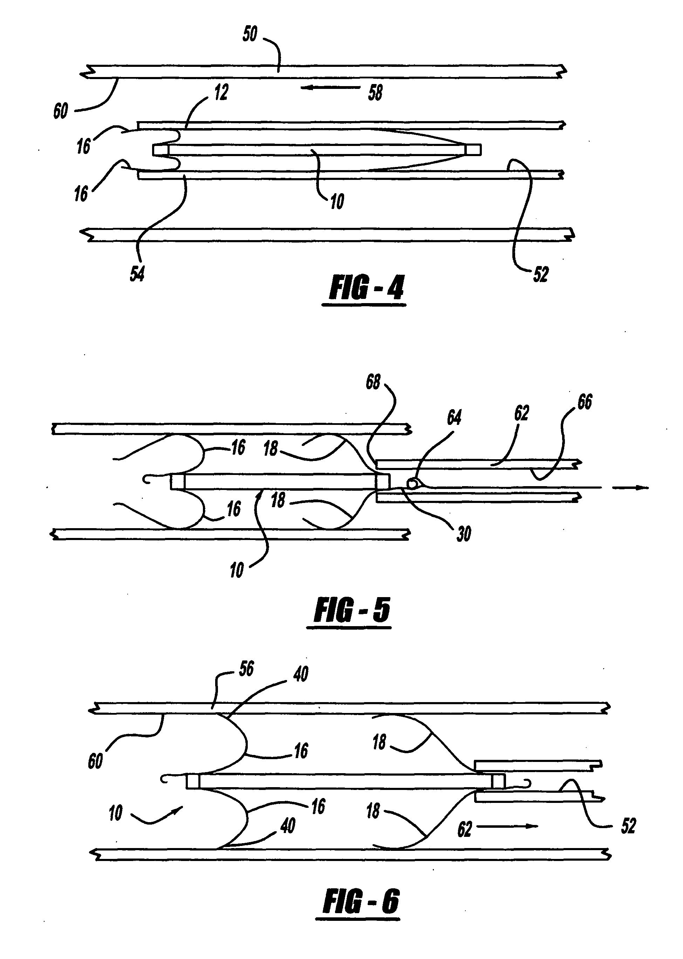

Emplacement of filter 10 is illustrated in Figure 4. As shown in Figure 4, delivery catheter 12 carrying radially compressed filter 10 in lumen 52 at distal end 54 of catheter 12 is inserted along body vessel 56 in a patient until distal end 54 is near the desired site for treatment. Catheter 12 may be inserted using either a femoral approach or a jugular approach. A push wire (not shown in the Figures) may be used to eject filter from lumen 52 by pushing filter 10 in the direction indicated by arrow 58. As filter 10 is ejected, arms 16 expand radially outwardly until bend radius 40 of arm 16 contacts wall 60 of vessel 56. After arms 16 are deployed, delivery catheter 12 is withdrawn slightly in the direction indicated in Figure 4 by arrow 62 whereupon filter 10 is completely ejected from lumen 52 and arms 18 expand into contacting relationship with wall 60 of vessel 56 and assume an operative filtering position within vessel 56 as is best illustrated in Figure 5.

Once emplaced in a body passage such as a vein and now referring to Figure 2, filter 10 has struts 16 and 18 with ends 38 positioned proximal to wall 60 of vessel 56 but spaced therefrom so as to avoid piercing into vessel wall 60 and also to minimize any encapsulation by tissue growth. In operation, filter 10 provides a first filter basket 20 and a second filter basket 22 with basket openings facing each other so that the particular orientation of filter 10 with respect to flow of blood is not important. Furthermore, filter 10 as retrieval hooks 26 and 20 at opposite ends so that filter 10 can be retrieved from either direction.

While filter 10 is suitable for permanent placement in vessel 56, filter 10 may be readily removed or retrieved from vessel 56 if retrieval is desired. As is shown in Figures 5 and 6, the retrieval of filter 10 may be accomplished by means of a retrieval catheter 62 which may be inserted from either the femoral or jugular direction. After snaring retrieval hook 30 with snare 64 of retrieval catheter 62, filter 10 is drawn into lumen 66 of retrieval catheter 62 as shown in Figure 5 whereupon struts 18 come into contact with distal end 68 of retrieval catheter 62 which contacting relationship urges the loose ends of struts 18 radially inwardly as shown in Figure 6. Pulling of filter 10 rightward as shown in Figure 5 results in struts 16 reversely bending to the positions shown in Figure 6. Further pullinjg of filter 10 into lumen 66 results in distal end 68 of retrieval catheter 62 coming into contact with struts 16 to urge struts 16 radially inwardly. Struts 16 thus are inverted as they are bent by the force exerted by end 68 of retrieval catheter 62 in a leftward direction as viewed in the figures and are ultimately bent radially inwardly as shown in Figure 6. Because struts 16 and 18 have free ends 38, they may be bent as shown in the figures to radially collapsed positions which can be compressed within lumen 66 for ready withdrawal from vessel 56.

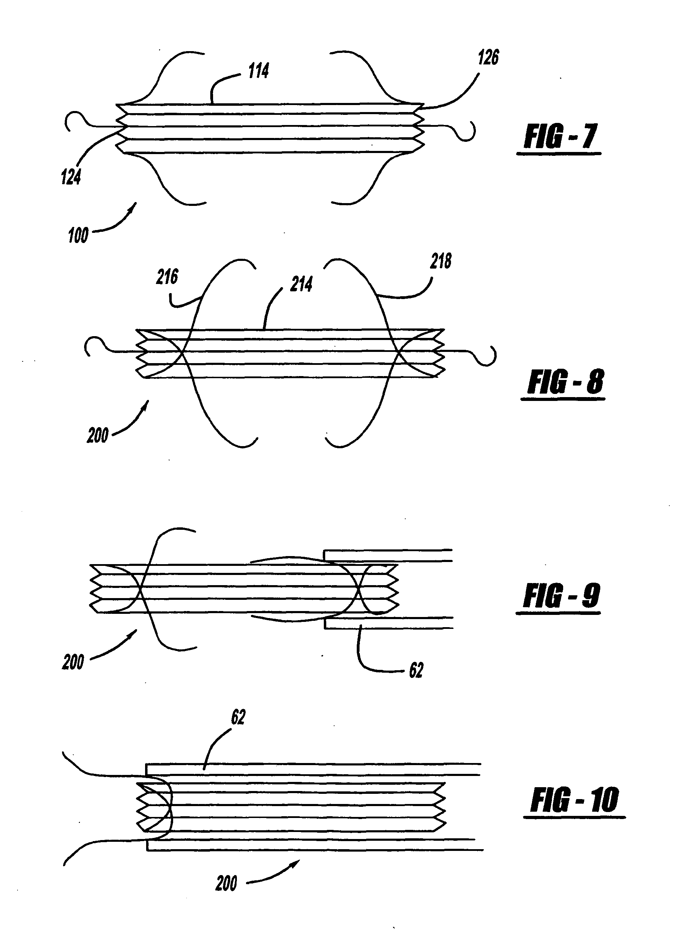

Now referring to Figure 7, an alternative preferred embodiment of a filter of the present invention is shown and indicated generally by numeral 100. Filter 100 is similar in construction to filter 10. But has a spine 114 with serpentine end rings 124 and 126. Both spine 114 and serpentine end rings 124 and 126 compress radially for insertion into the lumen of a delivery catheter but expand to a relatively greater radius when deployed as shown in Figure 8. Filter 100 is well adapted to be made from a tubular material by cutting the respective parts therefrom. Retrieval of filter 100 is analogous to retrieval of filter 10.

Figure 8 shows another alternative embodiment of a filter of the present invention which is indicated generally by the numeral 200. Filter 200 is a cross-over design wherein struts 216 and 218 extend from spine 214 initially in a generally circumferential direction. Figure 9 shows filter 200 in a deployed state. Filter 200 is substantially equivalent to filter 10 in filtering efficiency. And retrieval of filter 200 can be done in a manner analogous to retrieval of filter 10. However, during retrieval, the somewhat circumferential orientation of struts 216 and 218 is advantageous because retrieval into the lumen of a retrieval catheter can be achieved with a greater bending radius of the struts as is illustrated in Figure 9 and 10. This feature allows use of a lumen with a small diameter because the cross-over design minimizes the size of the filter during retrieval. The filter size of filter 10 during retrieval is determined by the bend radius of struts 16. The filter size of filter 200 during retrieval is reduced by the difference between the bend radius of the strut and the diameter of the end ring.

A filter made in accordance with the present invention is retrievable at any time following implantation and may be retrieved from either femoral or jugular approaches. The cross-over design of the preferred embodiment shown in Figure 8 further reduces the size of the filter during retrieval. The filter has the clot capture efficiency of a double-basket filter.

While preferred embodiments of the present invention have been specifically described above, it will be appreciated by those skilled in the art that the present invention is subject to variations and modifications. For example, the filter may be cut from a single tube and have end rings which are integral, i.e., one piece, with the spine and struts. The end rings may be tubular or cut into a serpentine pattern to allow the end rings to expandable to a larger diameter and subsequently compressible to a smaller diameter. This feature allows for very low profile filters when in the compressed state- an obvious advantage for insertion and removal. Of course, the filter may be tube-based or wire based or a combination.

## Claims

1. A medical filter (10, 100, 200) for placement inside a body passage to treat a patient, comprising:
in a radially compressed state, a longitudinally extending spine (14, 114, 214) having a plurality of struts (16, 216) secured at one longitudinal end of said spine (14, 114, 214) and a plurality of struts (18, 21) secured at an opposite longitudinal end of said spine (14, 114, 214), each of said struts (16, 18, 216, 218) being biased in a radially outward direction;
in a radially expanded state, each of said struts (16, 18, 216, 218) extending in a direction generally radially outwardly and axially from said spine (14, 114, 214) and each of said struts (16, 18, 216, 218) having a first bend (34) proximate to said spine (14, 114, 214) in a direction away from said spine (14, 114, 214) and a second bend (40) at each free end portion (34) in a direction toward said spine (14, 114, 214)
**characterized in that**, in the radially compressed state, each of said struts (16, 18, 216, 218) extends generally longitudinally along said spine (14, 114, 214).

2. A medical filter (10, 100, 200) as set forth in claim 1, wherein each strut (16, 18, 216, 218) is a wire.

3. A medical filter (10, 100, 200) as set forth in claim 1, wherein each strut (16, 18, 216, 218) extends from said spine in circumferential, radial and axial directions when in a radially expanded state.

4. A medical filter (10, 100, 200) as set forth in claim 1, wherein each end of said spine (14, 114, 214) carries a retrieval hook (26, 30).

5. A medical filter (10, 100, 200) as set forth in claim 1, wherein a ring (24, 28, 124, 126) is at each longitudinal end of said spine and said struts are secured to said rings.

6. A medical filter (10, 100, 200) as set forth in claim 1, wherein said spine (14, 114, 214) and said struts (16, 18, 216, 218) are comprised of wires.

7. A medical filter (10, 100, 200) as set forth in claim 1, wherein said spine (14, 114, 214) and said struts (16, 18, 216, 218) are comprised of flat wires.

8. A medical filter (100, 200) as set forth in claim 1, wherein said end rings (124, 126) have serpentine cuts therein wherefore said rings can be expanded and contracted to greater and lesser diameters.

9. A medical filter (10, 100, 200) as set forth in claim 1, wherein said filter (10, 100, 200) has an integral, one-piece construction.

10. A medical filter according to any one of the preceding claims wherein, in the radially expanded state, said plurality of struts (16, 216) at one longitudinal end of the spine (14, 114, 214) form a first filter basket (20), said plurality of struts (18, 218) at the opposite longitudinal end form a second filter basket (22), and said first basket (20) and said second filter basket (22) have basket openings facing each other.

## Patentansprüche

1. Medizinischer Filter (10, 100, 200) zur Anordnung in einem Körperkanal zum Behandeln eines Patienten, umfassend:
in einem radial komprimierten Zustand, einen longitudinal verlaufenden Dorn (14, 114, 214) mit einer Vielzahl von Streben (16, 216), die an einem longitudinalen Ende des Doms (14, 114, 214) befestigt sind, und einer Vielzahl von Streben (18, 21), die an einem gegenüberliegenden longitudinalen Ende des Doms (14, 114, 214) befestigt sind, wobei jede der Streben (16, 18, 216, 218) in einer radial äußeren Richtung vorgespannt ist;
wobei in einem radial expandierten Zustand jede der Streben (16, 18, 216, 218) in einer Richtung allgemein radial nach außen und axial von dem Dom (14, 114, 214) verläuft und jede der Streben (16, 18, 216, 218) eine erste Krümmung (34) in der Nähe des Doms (14, 114, 214) in einer Richtung von dem Dom (14, 114, 214) weg und eine zweite Krümmung (40) an jedem freien Endabschnitt (34) in einer Richtung zum Dom (14, 114, 214) aufweist,
**dadurch gekennzeichnet, dass** in dem radial komprimierten Zustand jede der Streben (16, 18, 216, 218) allgemein longitudinal entlang des Doms (14, 11,4 214) verläuft.

2. Medizinischer Filter (10, 100, 200) nach Anspruch 1, **dadurch gekennzeichnet, dass** jede Strebe (16, 18, 216, 218) ein Draht ist.

3. Medizinischer Filter (10, 100, 200) nach Anspruch 1, **dadurch gekennzeichnet, dass** jede Strebe (16, 18, 216, 218) von dem Dom in umlaufenden, radialen und axialen Richtungen in einem radial expandierten Zustand verläuft.

4. Medizinischer Filter (10, 100, 200) nach Anspruch 1, **dadurch gekennzeichnet, dass** jedes Ende des Doms (14, 114, 214) einen Zurückholhaken (26, 30) trägt.

5. Medizinischer Filter (10, 100, 200) nach Anspruch 1, **dadurch gekennzeichnet, dass** sich ein Ring (24, 28, 124, 126) an jedem longitudinalen Ende des Doms befindet und die Streben an den Ringen befestigt sind.

6. Medizinischer Filter (10, 100, 200) nach Anspruch 1, **dadurch gekennzeichnet, dass** der Dom (14, 114, 214) und die Streben (16, 18, 216, 218) aus Drähten bestehen.

7. Medizinischer Filter (10, 100, 200) nach Anspruch 1, **dadurch gekennzeichnet, dass** der Dom (14, 114, 214) und die Streben (16, 18, 216, 218) aus Flachdrähten bestehen.

8. Medizinischer Filter (10, 100, 200) nach Anspruch 1, **dadurch gekennzeichnet, dass** die Endringe (124, 126) schlangenförmige Einschnitte aufweisen, wodurch die Ringe auf größere Durchmesser aufgeweitet und auf geringere Durchmesser zusammengezogen werden können.

9. Medizinischer Filter (10, 100, 200) nach Anspruch 1, **dadurch gekennzeichnet, dass** der Filter (10, 100, 200) eine integrale, einstückige Konstruktion aufweist.

10. Medizinischer Filter nach einem der vorangehenden Ansprüche, **dadurch gekennzeichnet, dass** in dem radial expandierten Zustand die Vielzahl von Streben (16, 216) an einem longitudinalen Ende des Doms (14, 114, 214) einen ersten Filterkorb (20) bildet, die Vielzahl von Streben (18, 218) an dem gegenüberliegenden longitudinalen Ende einen zweiten Filterkorb (22) bildet und der erste Filterkorb (20) und der zweite Filterkorb (22) Korböffnungen aufweisen, die einander zugewandt sind.

## Revendications

1. Filtre médical (10, 100, 200) destiné à être placé à l'intérieur d'un passage d'un corps de manière à traiter un patient, comprenant :
→ dans un état comprimé de manière radiale, une colonne vertébrale qui s'étend de façon longitudinale (14, 114, 214) et qui présente une pluralité d'entretoises (16, 216) fixées au niveau d'une extrémité longitudinale de ladite colonne vertébrale (14, 114, 214) et une pluralité d'entretoises (18, 21) fixées au niveau d'une extrémité longitudinale opposée de ladite colonne vertébrale (14, 114, 214), chacune desdites entretoises (16, 18, 216, 218) étant orientée dans une direction vers l'extérieur de manière radiale ;
→ dans un état expansé de manière radiale, chacune desdites entretoises (16, 18, 216, 218) s'étendant dans une direction en général de manière radiale vers l'extérieur et de façon axiale à partir de ladite colonne vertébrale (14, 114, 214) et chacune desdites entretoises (16, 18, 216, 218) présentant un premier coude (34) proche de ladite colonne vertébrale (14, 114, 214) dans une direction en allant en s'éloignant de ladite colonne vertébrale (14, 114, 214) et un deuxième coude (40) au niveau de chaque partie d'extrémité libre (34) dans une direction en allant vers ladite colonne vertébrale (14, 114, 214) ;
**caractérisé en ce que**, dans l'état comprimé de manière radiale, chacune desdites entretoises (16, 18, 216, 218) s'étend en général de manière longitudinale le long de ladite colonne vertébrale (14, 114, 214).

2. Filtre médical (10, 100, 200) selon la revendication 1, dans lequel chaque entretoise (16, 18, 216, 218) est un fil.

3. Filtre médical (10, 100, 200) selon la revendication 1, dans lequel chaque entretoise (16, 18, 216, 218) s'étend à partir de ladite colonne vertébrale dans des directions circonférentielles, radiales et axiales dans un état expansé de manière radiale.

4. Filtre médical (10, 100, 200) selon la revendication 1, dans lequel chaque extrémité de ladite colonne vertébrale (14, 114, 214) porte un crochet de récupération.

5. Filtre médical (10, 100, 200) selon la revendication 1, dans lequel un anneau (24, 28, 124, 126) se situe au niveau de chaque extrémité longitudinale de ladite colonne vertébrale et lesdites entretoises sont fixées auxdits anneaux.

6. Filtre médical (10, 100, 200) selon la revendication 1, dans lequel ladite colonne vertébrale (14, 114, 214) et lesdites entretoises (16, 18, 216, 218) sont constituées par des fils.

7. Filtre médical (10, 100, 200) selon la revendication 1, dans lequel ladite colonne vertébrale (14, 114, 214) et lesdites entretoises (16, 18, 216, 218) sont constituées par des fils plats.

8. Filtre médical (100, 200) selon la revendication 1, dans lequel lesdits anneaux d'extrémité (124, 126) présentent à l'intérieur des découpes en serpentin grâce auxquelles lesdits anneaux peuvent être expansés et contractés de façon à prendre des diamètres plus grands et plus petits.

9. Filtre médical (10, 100, 200) selon la revendication 1, dans lequel ledit filtre (10, 100, 200) présente une construction d'une pièce.

10. Filtre médical selon l'une quelconque des revendications précédentes dans lequel, dans l'état expansé de manière radiale, ladite pluralité d'entretoises (16, 216) au niveau d'une extrémité longitudinale de la colonne vertébrale (14, 114, 214) forment un premier panier de filtre (20), ladite pluralité d'entretoises (18, 218) au niveau de l'extrémité longitudinale opposée forment un deuxième panier de filtre (22), et ledit premier panier de filtre (20) et ledit deuxième panier de filtre (22) présentent des ouvertures de panier qui se font face l'une à l'autre.
